# EUROPEAN PATENT SPECIFICATION

(11) **EP 0 394 434 B1**
(45) Date of publication and mention of the grant of the patent: **28.05.1997**
(21) Application number: 89912717.9
(22) Date of filing: 17.10.1989
(51) Int. Cl.: C01B 39/48, C01G 17/00, C01G 49/00, B01J 29/70, B01J 29/04, C10G 47/16, C07C 4/06, C07C 5/41, C10G 11/05, C07C 211/38

(54) **NEW ZEOLITE SSZ-31**
NEUES ZEOLITH SSZ-31
NOUVEAU ZEOLITE SSZ-31

(30) Priority: 20.10.1988 US 260439
(43) Date of publication of application: 31.10.1990
(73) Proprietor: CHEVRON U.S.A. Inc., Philadelphia Pennsylvania 19103 (US)
(72) Inventor: ZONES, Stacey, I., San Francisco, CA 94122 (US); HARRIS, Thomas, V., Benicia, CA 94510 (US); RAINIS, Andrew, Walnut Creek, CA 94594 (US); SANTILLI, Donald, S., Larkspur, CA 94939 (US)
(74) Representative: Benedum, Ulrich Max, Dr.
(86) International application number: US8904638
(87) International publication number: WO9004567

(56) References cited:
- WO-A-89/04860
- WO-A-92/13045
- US-A- 3 328 251
- US-A- 4 397 827
- US-A- 4 423 021
- US-A- 4 536 282
- US-A- 4 544 538
- US-A- 4 665 110
- US-A- 4 665 248
- CHEMICAL ABSTRACTS 68 (1) 2619m GAVRILOV et al (1968)
- ANGEW. CHEM., VOL. 100 (1988), PAGES 232-233 by W.Hölderich et al.

## Description

The present invention relates to a zeolite having a mole ratio of an oxide selected from silicon oxide, germanium oxide and mixtures thereof to an oxide selected from aluminum oxide, gallium oxide, and mixtures thereof, greater than 100:1.

Natural and synthetic zeolitic crystalline aluminosilicates are useful as catalysts and adsorbents. These aluminosilicates have distinct crystal structures which are demonstrated by X-ray diffraction. The crystal structure defines cavities and pores which are characteristic of the different species. The adsorptive and catalytic properties of each crystalline aluminosilicate are determined in part by the dimensions of its pores and cavities. Thus, the utility of a particular zeolite in a particular application depends at least partly on its crystal structure.

Because of their unique molecular sieving characteristics, as well as their catalytic properties, crystalline aluminosilicates are especially useful in such applications as gas drying and separation and hydrocarbon conversion. Although many different crystalline aluminosilicates and silicates have been disclosed, there is a continuing need for new zeolites and silicates with desirable properties for gas separation and drying, hydrocarbon and chemical conversions, and other applications.

Crystalline aluminosilicates are usually prepared from aqueous reaction mixtures containing alkali or alkaline earth metal oxides, silica, and alumina. "Nitrogenous zeolites" have been prepared from reaction mixtures containing an organic templating agent, usually a nitrogen-containing organic cation. By varying the synthesis conditions and the composition of the reaction mixture, different zeolites can be formed using the same templating agent. Use of N,N,N-trimethyl cyclopentylammonium iodide in the preparation of Zeolite SSZ-15 molecular sieve is disclosed in U.S. Patent No. 4,610,584; use of 1-azoniaspiro [4.4] nonyl bromide and N,N,N-trimethyl neopentylammonium iodide in the preparation of a molecular sieve termed "Losod" is disclosed in Helv. Chim. Acta (1974); Vol. 57, page 1533 (W.Sieber and W. M. Meier); use of quinuclidinium compounds to prepare a zeolite termed "NU3" is disclosed in European Patent Publication No. 40016; use of 1,4-di(l-azoniabicyclo [2.2.2.]octane)-lower alkane compounds in the preparation of Zeolite SSZ-16 molecular sieve is disclosed in U.S. Patent No. 4,508,837; use of N,N,N-trialkyl-l-adamantamine in the preparation of Zeolite SSZ-13 molecular sieve is disclosed in U.S. Patent No. 4,544,538.

U.S. Patents No. 4,423,021 and 4,397,827 disclose a Zeolite ZSM-48 with a silicon oxide to aluminum oxide ratio greater than 100. ZSM-48 is a 10-ring zeolite having medium size pores and can be prepared from a reaction mixture containing a source of silica, a tetramethyl ammonium compound, C₂-C₁₂ alkylamine, and an alkali metal oxide, e.g. sodium, with or without a source of alumina, and water. Its composition in terms of moles is as follows: (005 to 5)N₂O (0.1 to 10)M_{Z}/ₙO : (0 to 4)Al₂O₃ : 100(SiO₂) wherein M is at least one cation having a valence n, N is a mixture of a C₂-C₁₂ alkylamine and a tetramethyl ammonium compound.

### SUMMARY OF THE INVENTION

We have prepared a family of crystalline aluminosilicate molecular sieves with unique properties, referred to herein as "Zeolite SSZ-31", or simply "SSZ-31", and have found a highly effective method for preparing SSZ-31.

SSZ-31 has a mole ratio of an oxide selected from silicon oxide, germanium oxide, and mixtures thereof to an oxide selected from aluminum oxide, gallium oxide, iron oxide, and mixtures thereof-greater than about 100:1 and having the X-ray diffraction lines of Table 1 below. The zeolite further has a composition, as synthesized and in the anhydrous state, in terms of mole ratios of oxides as follows: (0.1 to 10)Q₂O : (0.1 to 5.0) M₂O : W₂O₃ : (greater than 100)YO₂ wherein M is an alkali metal cation, W is selected from aluminum, gallium, iron, and mixtures thereof, Y is selected from silicon, germanium and mixtures thereof, and Q is a tricyclodecane quaternary ammonium ion. SSZ-31 zeolites can have a YO₂:W₂O₃ mole ratio greater than about 100:1 and can be made essentially alumina free. As prepared, the silica:alumina mole ratio is typically in the range of 100:1 to about 10,000:1. Higher mole ratios can be obtained by treating the zeolite with chelating agents or acids to extract aluminum from the zeolite lattice. The silica:alumina mole ratio can also be increased by using silicon and carbon halides and other similar compounds. Preferably, SSZ-31 is an aluminosilicate wherein W is aluminum and Y is silicon.

Lower ratios of silica to alumina may also be obtained by using methods which insert alumina into the crystalline network. For example, alumina insertion may occur by thermal treatment of the zeolite in combination with an alumina binder or dissolved source of alumina. Such procedures are described in the prior art, e.g., U.S. patents Nos. 4,559,315 and 4,550,092.

By "essentially alumina free" as used herein, refers to silicaceous crystalline molecular sieves wherein any alumina is present as an impurity in the starting materials but for the impurity would not be present in the silicate.

Our invention also involves a method for preparing SSZ-31 zeolites, comprising preparing an aqueous mixture containing sources of a tricyclodecane quaternary ammonium ion, an oxide selected from aluminum oxide, gallium oxide, iron oxide, and mixtures thereof, and an oxide selected from silicon oxide, germanium oxide, and mixtures thereof, and having a composition, in terms of mole ratios of oxides, falling within the following ranges: YO₂/W₂O₃, 100:1 to infinity (essentially pure YO₂); wherein Y is selected from silicon, germanium, and mixtures thereof, W is selected from aluminum, gallium, iron, and mixtures thereof, and Q is a tricyclodecane quaternary ammonium ion; maintaining the mixture at a temperature of at least 100°C until the crystals of said zeolite are formed; and recovering said crystals.

### DETAILED DESCRIPTION OF THE INVENTION

SSZ-31 zeolites, as synthesized, have a crystalline structure whose X-ray powder diffraction pattern shows the following characteristic lines:

**Table 1**

| Principal Lines for SSZ-31 | | |
|---|---|---|
| 2θ | d/n | I/Iₒ |
| 6.10 | 14.49 | 6 |
| 7.38 | 11.98 | 30 |
| 8.18 | 10.81 | 11 |
| 20.30 | 4.37 | 15 |
| 21.12 | 4.21 | 69 |
| 22.25 | 3.99 | 100 |
| 24.73 | 3.60 | 23 |
| 30.90 | 2.89 | 11 |

Typical SSZ-31 aluminosilicate zeolites as prepared have the X-ray diffraction pattern of Tables 2 and 3 below.

The X-ray powder diffraction patterns were determined by standard techniques. The radiation was the K alpha/doublet of copper and a scintillation counter spectrometer with a stripchart pen recorder was used. The peak heights I and the positions, as a function of 2θ where θ is the Bragg angle, were read from the spectrometer chart. From these measured values, the relative intensities, 100I/I₀, where I₀ is the intensity of the strongest line or peak, and d, the interplanar spacing in Angstroms corresponding to the recorded lines, can be calculated. The X-ray diffraction pattern of Table 1 is characteristic of SSZ-31 zeolites. The zeolite produced by exchanging the metal or other cations present in the zeolite with various other cations yields substantially the same diffraction pattern although there can be minor shifts in interplanar spacing and minor variations in relative intensity. Minor variations in the diffraction pattern can also result from variations in the organic compound used in the preparation and from variations in the silicatoalumina mole ratio from sample to sample. Calcination, and the method of carrying out this step, can also cause minor shifts in the X-ray diffraction pattern. Notwithstanding these minor perturbations, the basic crystal lattice structure remains unchanged.

SSZ-31 zeolites can be suitably prepared from an aqueous solution containing sources of an alkali metal oxide, a tricyclodecane quaternary ammonium ion, an oxide of aluminum, gallium, iron, or mixtures thereof, and an oxide of silicon or germanium, or mixture of the two. The reaction mixture should have a composition in terms of mole ratios falling within the following ranges:

| | Broad | Preferred |
|---|---|---|
| YO₂/W₂O₃ | 100-∞ | 200-∞ |
| OH⁻/YO₂ | 0.10-1.0 | 0.20-0.50 |
| Q/YO₂ | 0.05-0.50 | 0.10-0.40 |
| M⁺/YO₂ | 0.05-0.30 | 0.05-0.15 |
| H₂O/YO₂ | 10-300 | 25-60 |
| Q/Q+M⁺ | 0.30-0.80 | 0.40-0.75 |

wherein Q is a tricyclodecane quaternary ammonium ion, Y is silicon, germanium or both, and W is aluminum, gallium, iron, or mixtures thereof. M is an alkali metal, preferably sodium or potassium. The organic tricyclodecane compound which acts as a source of the quaternary ammonium ion employed can provide hydroxide ion.

When using the quaternary ammonium hydroxide compound as a template, it has also been found that purer forms of SSZ-31 are prepared when there is an excess of tricyclodecane compound present relative to the amount of alkali metal hydroxide and that when the OH⁻/SiO₂ molar ratio is greater than 0.40, then M⁺/SiO₂ molar ratio should be less than 0.20.

The quaternary ammonium ion component Q, of the crystallization mixture, is derived from a [5.2.1.0] tricyclodecane quaternary ammonium compound with the nitrogen at the eight position of the ring system. Preferably, the quaternary ammonium ion is derived from a compound of the Formula (1) 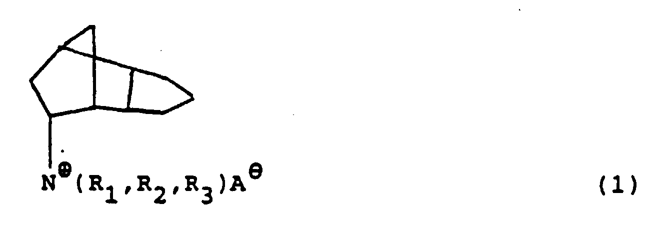 wherein each of R₁, R₂ and R₃ independently is lower alkyl and most preferably methyl; and A^{θ} is an anion which is not detrimental to the formation of the zeolite.

The tricyclodecane quaternary ammonium compounds of the Formula (1) above are prepared by methods known in the art. For example, compounds of the Formula (1) wherein A^{θ} is a halide may be prepared by reacting an N,N-di(lower)alkyl-8-amino tricyclo [5.2.1.0] decane compound of the Formula (2) 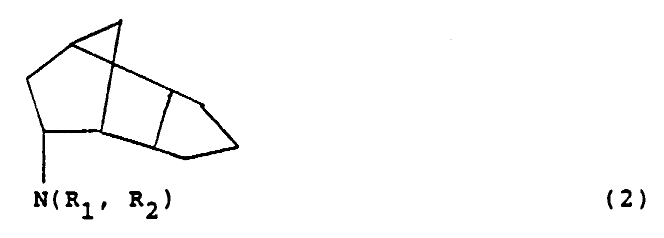 wherein each of R₁ and R₂ independently is lower alkyl, with a lower alkyl halide, in a solvent such as ethyl acetate. The halide anion may be ion exchanged to obtain other anions such as hydroxide, acetate, sulfate, carboxydate, and the like. The N,N-di(lower)alkyl-8-amino tricyclo [5.2.1.0] decane of the Formula (2) above may be prepared by reacting 8-ketotricyclo [5.2.1.0] decane with a lower dialkyl formamide in the presence of formic acid at a temperature in the range of 160°-195°C in a closed system. The reaction can be carried out for 10-50 hours, with the product recovered by partitioning between ether and a basic aqueous solution.

By "lower alkyl" is meant alkyl of from about 1 to 3 carbon atoms.

A^{⊖} is an anion which is not detrimental to the formation of the zeolite. Representative of the anions include halogen, e.g., fluoride, chloride, bromide and iodide, hydroxide, acetate, sulfate, carboxylate, etc. Hydroxide is the most preferred anion. It may be beneficial to ion-exchange, for example, the halide for hydroxide ion, thereby reducing or eliminating the alkali metal hydroxide quantity required.

The reaction mixture is prepared using standard zeolitic preparation techniques. Typical sources of aluminum oxide for the reaction mixture include aluminates, alumina, other zeolites, and aluminum compounds such as AlCl₃ and Al₂(SO₄)₃, and colloidal dispersions of alumina and alumina on silica, such as the Nalco product 1SJ612. Typical sources of silicon oxide include silicates, silica hydrogel, silicic acid, colloidal silica, tetraalkyl orthosilicates, and silica hydroxides. Gallium, iron, and germanium can be added in forms corresponding to their aluminum and silicon counterparts. Salts, particularly alkali metal halides such as sodium chloride, can be added to or formed in the reaction mixture. They are disclosed in the literature as aiding the crystallization of zeolites while preventing silica occlusion in the lattice.

The reaction mixture is maintained at an elevated temperature until the crystals of the zeolite are formed. The temperatures during the hydrothermal crystallization step are typically maintained from about 140°C to about 200°C, preferably from about 150°C to about 170°C and most preferably from about 155°C to about 165°C. The crystallization period is typically greater than 1 day and preferably from about 6 days to about 12 days.

The hydrothermal crystallization is conducted under pressure and usually in an autoclave so that the reaction mixture is subject to autogenous pressure. The reaction mixture can be stirred during crystallization.

Once the zeolite crystals have formed, the solid product is separated from the reaction mixture by standard mechanical separation techniques such as filtration. The crystals are waterwashed and then dried, e.g., at 90°C to 150°C for from 8 to 24 hours, to obtain the as synthesized, SSZ-31 zeolite crystals. The drying step can be performed at atmospheric or subatmospheric pressures.

During the hydrothermal crystallization step, the SSZ-31 crystals can be allowed to nucleate spontaneously from the reaction mixture. The reaction mixture can also be seeded with SSZ-31 crystals both to direct, and accelerate the crystallization, as well as to minimize the formation of undesired aluminosilicate contaminants.

The synthetic SSZ-31 zeolites can be used as synthesized or can be thermally treated (calcined). Usually, it is desirable to remove the alkali metal cation by ion exchange and replace it with hydrogen, ammonium, or any desired metal ion. The zeolite can be leached with chelating agents, e.g., EDTA or dilute acid solutions, to increase the silica:alumina mole ratio. The zeolite can also be steamed; steaming helps stabilize the crystalline lattice to attack from acids. The zeolite can be used in intimate combination with hydrogenating components, such as tungsten, vanadium, molybdenum, rhenium, nickel, cobalt, chromium, manganese, or a noble metal, such as palladium or platinum, for those applications in which a hydrogenation-dehydrogenation function is desired. Typical replacing cations can include metal cations, e.g., rare earth, Group IIA and Group VIII metals, as well as their mixtures. Of the replacing metallic cations, cations of metals such as rare earth, Mn, Ca, Mg, Zn, Cd, Pt, Pd, Ni, Co, Ti, Al, Sn, Fe and Co are particularly preferred.

The hydrogen, ammonium, and metal components can be exchanged into the zeolite. The zeolite can also be impregnated with the metals, or, the metals can be physically intimately admixed with the zeolite using standard methods known to the art. And, the metals can be occluded in the crystal lattice by having the desired metals present as ions in the reaction mixture from which the SSZ-31 zeolite is prepared.

Typical ion exchange techniques involve contacting the synthetic zeolite with a solution containing a salt of the desired replacing cation or cations. Although a wide variety of salts can be employed, chlorides and other halides, nitrates, and sulfates are particularly preferred. Representative ion exchange techniques are disclosed in a wide variety of patents including U.S. Nos. 3,140,249; 3,140,251; and 3,140,253. Ion exchange can take place either before or after the zeolite is calcined.

Following contact with the salt solution of the desired replacing cation, the zeolite is typically washed with water and dried at temperatures ranging from 65°C to about 315°C. After washing, the zeolite can be calcined in air or inert gas at temperatures ranging from about 200°C to 820°C for periods of time ranging from 1 to 48 hours, or more, to produce a catalytically active product especially useful in hydrocarbon conversion processes.

Regardless of the cations present in the synthesized form of the zeolite, the spatial arrangement of the atoms which form the basic crystal lattice of the zeolite remains essentially unchanged. The exchange of cations has little, if any, effect on the zeolite lattice structures.

The SSZ-31 aluminosilicate can be formed into a wide variety of physical shapes. Generally speaking, the zeolite can be in the form of a powder, a granule, or a molded product, such as extrudate having particle size sufficient to pass through a 2-mesh (Tyler) screen and be retained on a 400-mesh (Tyler) screen. In cases where the catalyst is molded, such as by extrusion with an inorganic binder, the aluminosilicate can be extruded before drying, or, dried or partially dried and then extruded. The zeolite can be composited with other materials resistant to the temperatures and other conditions employed in organic conversion processes. Such matrix materials include active and inactive materials and synthetic or naturally occurring zeolites as well as inorganic materials such as clays, silica and metal oxides. The latter may occur naturally or may be in the form of gelatinous precipitates, sols, or gels, including mixtures of silica and metal oxides. Use of an active material in conjunction with the synthetic zeolite, i.e., combined with it, tends to improve the conversion and selectivity of the catalyst in certain organic conversion processes. Inactive materials can suitably serve as diluents to control the amount of conversion in a given process so that products can be obtained economically without using other means for controlling the rate of reaction. Frequently, zeolite materials have been incorporated into naturally occurring clays, e.g., bentonite and kaolin. These materials, i.e., clays, oxides, etc., function, in part, as binders for the catalyst. It is desirable to provide a catalyst having good crush strength, because in petroleum refining the catalyst is often subjected to rough. handling. This tends to break the catalyst down into powders which cause problems in processing.

Naturally occurring clays which can be composited with the synthetic zeolites of this invention include the montmorillonite and kaolin families, which families include the subbentonites and the kaolins commonly known as Dixie, McNamee, Georgia and Florida clays or others in which the main mineral constituent is halloysite, kaolinite, dickite, nacrite, or anauxite. Fibrous clays such as sepiolite and attapulgite can also be used as supports. Such clays can be used in the raw state as originally mined or can be initially subjected to calcination, acid treatment or chemical modification.

In addition to the foregoing materials, the SSZ-31 zeolites can be composited with porous matrix materials and mixtures of matrix materials such as silica, alumina, titania, magnesia, silica:alumina, silica-magnesia, silica-zirconia, silica-thoria, silica-beryllia, silicatitania, titaniazirconia as well as ternary compositions such as silica-alumina-thoria, silica-alumina-zirconia, silica-alumina-magnesia and silica-magnesia-zirconia. The matrix can be in the form of a cogel.

The SSZ-31 zeolites can also be composited with other zeolites such as synthetic and natural faujasites (e.g., X and Y), erionites, and mordenites. They can also be composited with purely synthetic zeolites such as those of the ZSM series, the ICI series, or the large variety of Union Carbide synthetic sieves. The combination of zeolites can also be composited in a porous inorganic matrix.

SSZ-31 zeolites are useful in hydrocarbon conversion reactions. Hydrocarbon conversion reactions are chemical and catalytic processes in which carbon containing compounds are changed to different carbon containing compounds. Examples of hydrocarbon conversion reactions include catalytic cracking, hydrocracking, and olefin and aromatics formation reactions. The catalysts are useful in other petroleum refining and hydrocarbon conversion reactions such as isomerizing n-paraffins and naphthenes, polymerizing and oligomerizing olefinic or acetylenic compounds such as isobutylene and butene-1, reforming, alkylating, isomerizing polyalkyl substituted aromatics (e.g., ortho xylene), and disproportionating aromatics (e.g., toluene) to provide mixtures of benzene, xylenes and higher methylbenzenes. The SSZ-31 catalysts have high selectivity, and under hydrocarbon conversion conditions can provide a high percentage of desired products relative to total products.

SSZ-31 zeolites can be used in processing hydrocarbonaceous feedstocks. Hydrocarbonaceous feedstocks contain carbon compounds and can be from many different sources, such as virgin petroleum fractions, recycle petroleum fractions, shale oil, liquefied coal, tar sand oil, and, in general, can be any carbon containing fluid susceptible to zeolitic catalytic reactions. Depending on the type of processing the hydrocarbonaceous feed is to undergo, the feed can contain metal or be free of metals, it can also have high or low nitrogen or sulfur impurities. It can be appreciated, however, that in general processing will be more efficient (and the catalyst more active) the lower the metal, nitrogen, and sulfur content of the feedstock.

Using SSZ-31 catalyst which contains a hydrogenation promoter, heavy petroleum residual feedstocks, cyclic stocks and other hydrocrackate charge stocks can be hydrocracked at hydrocracking conditions including a temperature in the range of from 175°C to 485°C, molar ratios of hydrogen to hydrocarbon charge from 1 to 100, a pressure in the range of from 0.5 to 350 bar, and a liquid hourly space velocity (LHSV) in the range of from 0.1 to 30.

The hydrocracking catalysts contain an effective amount of at least one hydrogenation catalyst (component) of the type commonly employed in hydrocracking catalysts. The hydrogenation component is generally selected from the group of hydrogenation catalysts consisting of one or more metals of Group VIB and Group VIII, including the salts, complexes and solutions containing such. The hydrogenation catalyst is preferably selected from the group of metals, salts and complexes thereof of the group consisting of at least one of platinum, palladium, rhodium, iridium and mixtures thereof or the group consisting of at least one of nickel, molybdenum, cobalt, tungsten, titanium, chromium and mixtures thereof. Reference to the catalytically active metal or metals is intended to encompass such metal or metals in the elemental state or in some form such as an oxide, sulfide, halide, carboxylate and the like.

The hydrogenation catalyst is present in an effective amount to provide the hydrogenation function of the hydrocracking catalyst, and preferably in the range of from 0.05 to 25% by weight.

The catalyst may be employed in conjunction with traditional hydrocracking catalysts, e.g., any aluminosilicate heretofore employed as a component in hydrocracking catalysts. Representative of the zeolitic aluminosilicates disclosed heretofore as employable as component parts of hydrocracking catalysts are Zeolite Y (including steam stabilized, e.g., ultra-stable Y), Zeolite X, Zeolite beta (U.S. Patent No. 3,308,069), Zeolite ZK-20 (U.S. Patent No. 3,445,727), Zeolite ZSM-3 (U.S. Patent No. 3,415,736), faujasite, LZ-10 (U.K. Patent 2,014,970, June 9, 1982), ZSM-5-type zeolites, e.g., ZSM-5, ZSM-11, ZSM-12, ZSM-23, ZSM-35, ZSM-38, ZSM-48, crystalline silicates such as silicalite (U.S. Patent No. 4,061,724), erionite, mordenite, offretite, chabazite, FU-1-type zeolite, NU-type zeolites, LZ-210-type zeolite and mixtures thereof. Traditional cracking catalysts containing amounts of Na₂O less than about one percent by weight are generally preferred. The relative amounts of the SSZ-31 component and traditional hydrocracking component, if any, will depend at least in part, on the selected hydrocarbon feedstock and on the desired product distribution to be obtained therefrom, but in all instances an effective amount of SSZ-31 is employed. When a traditional hydrocracking catalyst (THC) component is employed the relative weight ratio of the THC to the SSZ-31 is generally between about 1:10 and about 500:1, desirably between about 1:10 and about 200:1, preferably between about 1:2 and about 50:1, and most preferably is between about 1:1 and about 20:1.

The hydrocracking catalysts are typically employed with an inorganic oxide matrix component which may be any of the inorganic oxide matrix components which have been employed heretofore in the formulation of hydrocracking catalysts including: amorphous catalytic inorganic oxides, e.g., catalytically active silicaaluminas, clays, silicas, aluminas, silicaaluminas, silicazirconias, silicamagnesias, aluminaborias, aluminatitanias and the like and mixtures thereof. The traditional hydrocracking catalyst and SSZ-31 may be mixed separately with the matrix component and then mixed or the THC component and SSZ-31 may be mixed and then formed with the matrix component.

SSZ-31 can be used to convert light straight run naphthas and similar mixtures to highly aromatic mixtures. Thus, normal and slightly branched chained hydrocarbons, preferably having a boiling range above about 40°C and less than about 200°C, can be converted to products having a substantial aromatics content by contacting the hydrocarbon feed with the zeolite at a temperature in the range of from about 400°C to 600°C, preferably 480°C-550°C at pressures ranging from atmospheric to 10 bar, and liquid hourly space velocities (LHSV) ranging from 0.1 to 15.

The conversion catalyst preferably contain a Group VIII metal compound to have sufficient activity for commercial use. By Group VIII metal compound as used herein is meant the metal itself or a compound thereof. The Group VIII noble metals and their compounds, platinum, palladium, and iridium, or combinations thereof can be used. The most preferred metal is platinum. The amount of Group VIII metal present in the conversion catalyst should be within the normal range of use in reforming catalysts, from about 0.05 to 2.0 weight percent, preferably 0.2 to 0.8 weight percent.

The zeolite/Group VIII metal conversion catalyst can be used with or without a binder or matrix. The preferred inorganic matrix, where one is used, is a silicabased binder such as Cab-O-Sil or Ludox. Other matrices such as magnesia and titania can be used. The preferred inorganic matrix is nonacidic.

It is critical to the selective production of aromatics in useful quantities that the conversion catalyst be substantially free of acidity, for example by poisoning the zeolite with a basic metal, e.g., alkali metal, compound. The zeolite is usually prepared from mixtures containing alkali metal hydroxides and thus have alkali metal contents of about 1-2 weight percent. These high levels of alkali metal, usually sodium or potassium, are unacceptable for most catalytic applications because they greatly deactivate the catalyst for cracking reactions. Usually, the alkali metal is removed to low levels by ion-exchange with hydrogen or ammonium ions. By alkali metal compound as used herein is meant elemental or ionic alkali metals or their basic compounds. Surprisingly, unless the zeolite itself is substantially free of acidity, the basic compound is required in the present process to direct the synthetic reactions to aromatics production.

The amount of alkali metal necessary to render the zeolite substantially free of acidity can be calculated using standard techniques based on the aluminum content of the zeolite. Under normal circumstances, the zeolite as prepared and without ion-exchange may contain sufficient alkali metal to neutralize the acidity of the catalyst. If a zeolite free of alkali metal is the starting material, alkali metal ions can be ion exchanged into the zeolite to substantially eliminate the acidity of the zeolite. An alkali metal content of about 100%, or greater, of the acid sites calculated on a molar basis is sufficient.

Where the basic metal content is less than 100% of the acid sites on a molar basis, the test described in U.S. Patent No. 4,347,394 which patent is incorporated totally herein by reference, can be used to determine if the zeolite is substantially free of acidity.

The preferred alkali metals are sodium and potassium. The zeolite itself can be substantially free of acidity only at very high silica:alumina mol ratios; by "zeolite consisting essentially of silica" is meant a zeolite which is substantially free of acidity without base poisoning.

Hydrocarbon cracking stocks can be catalytically cracked in the absence of hydrogen using SSZ-31 at liquid hourly space velocities from 0.5 to 50, temperatures from about 126.7°C (260°F) to 885°C (1625°F) and pressures from subatmospheric to several hundred atmospheres, typically from about atmospheric to about 5 atmospheres (0.1 - 0.5 MPa).

For this purpose, the SSZ-31 catalyst can be composited with mixtures of inorganic oxide supports as well as traditional cracking catalyst.

The catalyst may be employed in conjuction with traditional cracking catalysts, e.g., any aluminosilicate heretofore employed as a component in cracking catalyst. Representatives of the zeolitic aluminosilicates employable as component parts of cracking catalysts are Zeolite Y (including steam stabilized chemically modified, e.g., ultra-stable Y), Zeolite X, Zeolite beta (U.S. Patent No. 3,308,069), Zeolite ZK-20 (U.S. No. 3,445,727), Zeolite ZSM-3 (U.S. Patent No. 3,415,736), faujasite, LZ-10 (U.K. Patent 2,014,970, June 9, 1982) ZSM-5-type zeolytes, e,g., ZSM-5, ZSM-11, ZSM-12, ZSM-23, ZSM-35, ZSM-38, ZSM-48, crystalline silicates such as silicalite (U.S. Patent No. 4,061,724), erionite, mordenite, offretite, chabazite, FU-1-type zeolite, NU-type zeolites, LZ-210-type zeolite and mixtures thereof, Traditional cracking catalysts containing amounts of Na₂O less than about one percent by weight are generally preferred. The relative amounts of the SSZ-31 component and traditional cracking component, if any, will depend at least in part, on the selected hydrocarbon feed-stock and on the desired product distribution to be obtained therefrom, but in all instances an effective amount of SSZ-31 is employed. When a traditional cracking catalyst (TC) component is employed the relative weight ratio of the TC to the SSZ-31 is generally between about 1:10 and about 500:1, desirably between about 1:10 and about 200:1, preferably between about 1:2 and about 50:1, and most preferably is between about 1:1 and about 20:1.

The cracking catalysts are typically employed with an inorganic oxide matrix component which may be any of the inorganic oxide matrix components which have been employed heretofore in the formulation of FCC catalysts including: amorphous catalytic inorganic oxides, e.g., catalytically active silica-alumina, clays, silica, alumina, silica-alumina, silica-zirconia, silica-magnesia, alumina-boria, alumina-titania and the like and mixtures thereof. The traditional cracking component and SSZ-31 may be mixed separately with the matrix component and then mixed or the TC component and SSZ-31 may be mixed and then formed with the matrix component.

The mixture of a traditional cracking catalyst and SSZ-31 may be carried out in any manner which results in the coincident presence of such in contact with the crude oil feedstock under catalytic cracking conditions. For example, a catalyst may be employed containing the traditional cracking catalyst and an SSZ-31 in single catalyst particles or SSZ-31 with or without a matrix component may be added as a discrete component to a traditional cracking catalyst.

SSZ-31 can be used to dewax hydrocarbonaceous feeds by selectively converting waxy n-paraffins to non-waxy paraffins. The crystalline SSZ-31 zeolite, when used as a dewaxing catalyst, gives improved yields and viscosity index (VI) because it reduces pour point by a different mechanism than conventional dewaxing catalyst such as ZSM-5. The SSZ-31 dewaxing catalyst is selective in that it appears to isomerize normal and slightly branched chain paraffins and cycloparaffins without much cracking of highly branched paraffins. While the n-paraffins, slightly branched paraffins and cycloparaffins undergo some cracking or hydrocracking, the degree of cracking which occurs is, however, limited so that the gas yield is reduced thereby preserving the economic value of the feedstock. Many of the prior art catalysts crack both the highly branched as well as the normal paraffins to lighter products and gases. Because these lighter products are generally of lower value than the high molecular weight materials, it would obviously be desirable to limit the degree of cracking which takes place during the process.

SSZ-31 may be used to dewax a variety of feedstocks ranging from relatively light distillate fractions up to high boiling stocks such as whole crude petroleum, reduced crudes, vacuum tower residua, cycle oils, synthetic crudes (e.g., shale oils, tar sand oil, etc.), gas oils, vacuum gas oils, foots oils, and other heavy oils. The feedstock will normally be a C₁₀+ feedstock generally boiling above about 176.7°C (350°F) since lighter oils will usually be free of significant quantities of waxy components. However, the process is particularly useful with waxy distillate stocks such as middle distillate stocks including gas oils, kerosenes, and jet fuels, lubricating oil stocks, heating oils and other distillate fractions whose pour point and viscosity need to be maintained within certain specification limits.

Lubricating oil stocks will generally boil above 230°C (450°F), more usually above 315°C (600°F). Hydrocracked stocks are a convenient source of lubricating stocks of this kind and also of other distillate fractions since they normally contain significant amounts of waxy n-paraffins. The feedstock of the present process will normally be a C₁₀+ feedstock containing paraffins, olefins, naphthenes, aromatics and heterocyclic compounds and with a substantial proportion of higher molecular weight n-paraffins and slightly branched paraffins which contribute to the waxy nature of the feedstock.

The catalytic dewaxing conditions are dependent on large measure on the feed used and upon the desired pour point. Generally, the temperature will be between about 200°C and about 475°C, preferably between about 250°C and about 450°C. The pressure is typically between about 0.1 MPa (15 psig) and about 20.6 MPa (3000 psig), preferably between about 1.37 MPa (200 psig) and 20.6 MPa (3000 psig). The liquid hourly space velocity (LHSV) preferably will be from 0.1 to 20, preferably between about 0.2 and about 10.

Hydrogen is preferably present in the reaction zone during the catalytic dewaxing process. The hyd gen to feed ratio is typically between about 0.118 (500) and about 7.1 m³/l (30,000 SCF/bbl) (standard cubic feet per barrel), preferably about 2.51 (1000) to about 50.3 m³/l (20,000 SCF/bbl). Generally, hydrogen will be separated from the product and recycled to the reaction zone. Typical feedstocks include light gas oil, heavy gas oils and reduced crudes boiling about 350°F.

The SSZ-31 hydrodewaxing catalyst may optionally contain a metal component of the type commonly employed in dewaxing catalysts. The metal component may be selected from the group of metal catalysts consisting of one or more metals of Group VIB and Group VIII, including the salts, complexes and solutions containing such metals. The preferred metal is at least one of the group of metals, salts and complexes selected from the group consisting of at least one of platinum, palladium, rhodium, iridium and mixtures thereof or at least one from the group consisting of nickel, molybdenum, cobalt, tungsten, titanium, chromium and mixtures thereof. Reference to the catalytically active metal or metals is intended to encompass such metal or metals in the elemental state or in some form such as an oxide, sulfide halide, carboxylate and the like.

The metal component is present in an amount to provide an effective hydrodewaxing catalyst preferably in the range of from about 0.05 to 5% by weight.

The SSZ-31 hydrodewaxing catalyst may be used alone or in conjunction with intermediate-pore (or medium-pore) molecular sieves. These intermediate-pore molecular sieves are shape selective in that they have a pore size which admits straight-chain n-paraffins either alone or with only slightly branched-chain paraffins but which exclude more highly branched materials and cycloaliphatics. Molecular sieves such as ZSM-5, ZSM-11, ZSM-12, ZSM-22, ZSM-23 and SAPO-11 are suitable for this purpose.

The intermediate-pore molecular sieves may be combined with the SSZ-31 or the isomerization dewaxing step using SSZ-31 may be followed by a separate selective dewaxing step using the intermediate-pore molecular sieve.

The relative amounts of the SSZ-31 component and shape selective intermediate-pore molecular sieve component, if any, will depend at least in part, on the selected hydrocarbon feedstock and on the desired product distribution to be obtained therefrom, but in all instances an effective amount of SSZ-31 is employed. When a shape selective molecular sieve component is employed the relative weight ratio of the shape selective molecular sieve to the SSZ-31 is generally between about 10:1 and about 1:500, desirably between about 10:1 and about 1:200, preferably between about 2:1 and about 1:50, and most preferably is between about 1:1 and about 1:20.

The conversion of hydrocarbonaceous feeds can take place in any convenient mode, for example, in fluidized bed, moving bed, or fixed bed reactors depending on the types of process desired. The formulation of the catalyst particles will vary depending on the conversion process and method of operation.

Other reactions which can be performed using the catalyst of this invention containing a metal, e.g., platinum, include hydrogenationdehydrogenation reactions, denitrogenation and desulfurization reactions.

SSZ-31 can also be used as an adsorbent, as a filler in paper, paint, and toothpastes, and as a water-softening agent in detergents.

The following examples illustrate the preparation of SSZ-31.

### EXAMPLES

### Example 1

### Preparation of N,N,N-Trimethyl-8-Ammonium Tricyclo[5.2.1.0] decane Hydroxide (template)

Five (5) grams of 8-ketotricyclo [5.2.1.0] decane (Aldrich Chemical Co.) was mixed with 2.63 g of formic acid (88%) and 4.5 g of dimethylformamide. The mixture was then heated in a pressure vessel for 16 hours at 190°C. Care should be taken to anticipate the increase in pressure the reaction experiences due to CO₂ evolution. The reaction was conveniently carried out in a Parr 4748 reactor with teflon liner. The workup consists of extracting N,N-dimethyl-8-amino tricyclo[5.2.1.0] decane from a basic (pH=12) aqueous solution with diethyl ether. The various extracts were dried with Na₂SO₄, the solvent removed and the product taken up in ethyl acetate. An excess of methyl iodide was added to a cooled solution which was then stirred at room temperature for several days. The crystals were collected and washed with diethyl ether to give N,N,N-trimethyl-8-ammonium tricyclo[5.2.1.0] decane iodide. The product has a melting point of 270-272°C and the elemental analyses and proton NMR are consistent with the expected structure. The vacuum-dried iodide salt was then ion-exchanged with ion-exchange resin AG 1x8 (in molar excess) to the hydroxide form. The exchange was performed over a column or more preferably by overnight stirring of the resin beads and the iodide salt in an aqueous solution designed to give about a 0.5 molar solution of the organic hydroxide.

### Example 2

1.5 Millimoles of the template from Example 1 were mixed with 0.035 g of NaOH (solid) in 7.5 ml H₂O. 0.60 Gram of Cabosil M5 was stirred into the solution. The mixture was heated in a Parr 4745 reactor at 150°C and without agitation for 20 days. The contents of the reactor were filtered, washed with distilled water, dried at 100°C and analyzed by X-ray diffraction. The product was found to be the novel structure SSZ-31. The pattern is tabulated in Table 2 below.

**Table 2**

| 2θ | d/n | I/Iₒ |
|---|---|---|
| 4.26 | 20.7 | 5 |
| 6.10 | 14.49 | 6 |
| 7.36 | 12.01 | 30 |
| 8.18 | 10.81 | 11 |
| | | |
| 10.72 | 8.25 | 1 |
| 12.03 | 7.36 | 1 |
| 14.33 | 6.18 | 1 |
| 14.71 | 6.02 | 1 |
| 15.91 | 5.57 | 2 |
| 17.46 | 5.08 | 7 |
| 18.44 | 4.811 | 9 |
| 20.30 | 4.374 | 15 |
| 21.12 | 4.206 | 69 |
| 21.38 | 4.156 | 9 |
| 22.24 | 3.997 | 100 |
| 22.68 | 3.921 | 7 |
| | | |
| 24.73 | 3.600 | 23 |
| 25.19 | 3.535 | 11 |
| 25.70 | 3.466 | 5 |
| | | |
| 26.70 | 3.339 | 9 |
| 27.20 | 3.278 | 5 |
| 27.70 | 3.220 | 5 |
| 28.18 | 3.167 | 2 |
| 28.77 | 3.103 | 4 |
| 29.00 | 3.079 | 3 |
| | | |
| 29.50 | 3.028 | 2 |
| 29.82 | 2.996 | 5 |
| | | |
| 30.56 | 2.925 | 2 |
| 30.90 | 2.894 | 11 |
| 32.16 | 2.783 | 5 |
| 32.76 | 2.734 | 6 |

### Example 3

The same reaction mixture of Example 2 was formed again. A Parr 4745 reactor was used but this time it was loaded onto a rotating (30 rpm) spit of a Blue M oven which was rotated at 30 RPM. The tumbling reactors were heated at 160°C for 6 days. The analogous work-up and analysis produced a crystalline SSZ-31.

### Example 4

2.25 Millimoles of template were mixed with 0.075 g of NaOH (solid) and 12 ml of H₂O. 0.90 Gram of Cabosil were added and the reaction was run as in Example 3 except the Na/SiO₂ ratio had been increased. After 11 days of reaction, the product was mostly SSZ-31 but there was also some Kenyaiite and tridymite impurity.

### Example 5

The same experiment as in Example 4 was repeated with the following few changes. NaOH was replaced by 0.09 g of KOH (solid) and the reaction was run at 150°C and 0 RPM (no stirring) and required 22 days to crystallize. The product was SSZ-31 with a small amount of amorphous material.

### Example 6

Example 5 was repeated. However, the reaction was seeded with the product of Example 4. After 10 days at 160°C but without stirring the product was SSZ-31 with a small impurity of Kenyaiite. This run demonstrates that crystallization, in the absence of stirring, can be made faster by the use of seed crystals.

### Example 7

(a) 5 Millimoles of the template of Example 1 and 0.06 g NaOH(s) were mixed in 11.8 ml H₂O. 0.90 Gram Cabosil was stirred in to produce a homogeneous solution. 0.19 Gram of Nalco iSJ 612 (26% SiO₂, 4% Al₂O₃) was added with stirring and several milligrams of seed crystals were also added. The sealed reaction was carried out at 160°C, 39 rpm, and over 10 days. The crystalline product was determined to be a very broadlined version of SSZ-31.
(b) When the same reaction was run without seed crystals and at 30 rpm, crystallization of SSZ-31 required 16 days.

### Example 8

The same experiment as Example 7 has repeated, except the source of aluminum was 0.05 g Y zeolite (SK-40). Seeds of SSZ-31 were once again added. After 10 days at 160°C and 30 rpm, the product had a broadlined version of SSZ-31 although not as broadened as in Example 7.

### Example 9

The crystalline products of Examples 2 and 4 were subjected to calcination as follows. The samples were heated in a muffle furnace from room temperature up to 540°C at a steadily increasing rate over a 7-hour period. The samples were maintained at 540°C for four more hours and then taken up to 600°C for an additional four hours. A 50/50 mixture of air and nitrogen was passed over the zeolite at a rate of 0.5664 m³ (20 standard cubic feet) per minute during heating. The calcined product of Example 2 had the X-ray diffraction lines indicated in Table 3 below.

**Table 3**

| Calcined SSZ-31 | | |
|---|---|---|
| 2θ | d/n | 100 x I/Iₒ |
| 5.05 | 17.5 | 2 |
| 6.10 | 14.49 | 27 |
| 7.39 | 11.96 | 96 |
| 8.19 | 10.80 | 43 |
| 10.35 | 8.55 | 1 |
| 10.81 | 8.18 | 6 |
| 12.20 | 7.25 | 2 |
| 14.45 | 6.13 | 14 |
| 14.84 | 5.97 | 9 |
| 16.00 | 5.54 | 1 |
| 17.54 | 5.06 | 5 |
| 18.46 | 4.806 | 6 |
| 20.37 | 4.360 | 13 |
| 21.10 | 4.210 | 64 |
| 21.53 | 4.127 | 4 |
| 22.40 | 3.969 | 100 |
| | | |
| 23.78 | 3.742 | 1 |
| 24.85 | 3.583 | 14 |
| 25.20 | 3.534 | 4 |
| | | |
| 26.20 | 3.401 | 14 |
| 26.80 | 3.326 | 6 |
| | | |
| 27.70 | 3.220 | 2 |
| 28.20 | 3.164 | 1 |
| | | |
| 28.95 | 3.084 | 3 |
| 29.18 | 3.060 | 3 |
| | | |
| 29.83 | 2.995 | 3 |
| 30.00 | 2.979 | 3 |
| | | |
| 31.00 | 2.885 | 7 |
| 32.32 | 2.770 | 3 |
| 32.86 | 2.726 | 3 |

### Example 10

Ion-exchange of the calcined materials from Example 9 was carried out using NH₄NO₃ to convert the zeolites from Na form to NH₄ and then eventually to the H form. Typically, the same mass of NH₄NO₃ as zeolite was slurried into H₂O at ratio of 50/1 H₂O to zeolite. The exchange solution was heated at 100°C for two hours and then filtered. This process was repeated four times. Finally, after the last exchange, the zeolite was washed several times with H₂O and dried. A repeat calcination as in Example 9 was carried out but without the final treatment at 600°C. This produces the H form of the zeolites. The surface area for this material was 300 m²/g. The micro pore volume was 0.12 cm³g as determined by the BET method with N₂ as absorbate.

### Example 11

The all-silica version of SSZ-31 was evaluated as a reforming catalyst. The zeolite powder was impregnated with Pt(NH₃)₄^{·}2NO₃ to give 0.7 wt % Pt. The material was calcined up to 600°F in air and maintained at this temperature for three hours. The powder was pelletized on a Carver press at 1000 psi and broken and meshed to 24-40.

The catalyst was evaluated at 510°C (950°F) in hydrogen under the following conditions:
Pressure = 1.38 MPa (200 psig)
H₂/HC = 6.4
WHSV = 6
Temp. = 510°C (950°F)

The feed was an iC₇ mixture (Philips Petroleum Company)

| | Feed | Product 1.5 Hours Onstream, % |
|---|---|---|
| Conversion, % | | 36 |
| Toluene | 0.52 | 7.10 |
| C₅-C₈ Octane | 63.7 | 69.7 |

### Example 12

The product of Example 7(a) was. treated as in Examples 9 and 10. This catalyst now contained acidity due to aluminum incorporation. Two back ion-exchanges with KNO₃ were performed and the catalyst was calcined to 537°C (1000°F). Next, a reforming catalyst was prepared as in Example 11. The catalyst was evaluated under the following conditions:
Pressure = 1.38 MPa (200 psig)
H₂/HC = 6.4
WHSV = 6
Temp. = 427°C (800°F)

The feed has an iC₇ mixture (Philips Petroleum Co.). The data for the run is given in Table 4. After 23 hours on stream, the temperature was raised to 482°C (900°F) and this data also appears in the Table. By comparison with Example 11, the incorporation of aluminum into the zeolite gives a more active reforming catalyst.

**TABLE 4**

| Time | 0.5 hrs. | 1 h (after 23 h at 427°C (800°F)) |
|---|---|---|
| Temp. | 427°C (800°F) | 482°C (900°F) |
| Conversion | 19.4% | 35.6% |
| Aromatization Select. | 43.7% | 55.6% |
| Toluene in Product | 7.82% | 18.93% |
| % Toluene in C₅+ aromatics | 92% | 96% |
| C₅-C₈ RON | 67.2 | 72.7 |

### Example 13

The product of Example 7(b) was treated as in Examples 9 and 10. Next, the zeolite powder was pelletized in a Carver press at 6.8 MPa (1000 psi). The pellets were broken up and meshed to 24-40 size. 0.35 Gram of the hydrogen form was loaded into a 3/8-in. stainless steel tube with alumina packed on either side of the bed. The bed was heated in a Lindberg furnace and Helium (10 cm³/min) was introduced into the reactor. The catalyst was heated to 371°C (700°F). Once temperature equilibration was achieved, a 50/50 w/w feed of n-hexane/3 methylpentane was introduced into the reactor at WHSV = 0.68. The products were sampled on line by capillary G.C. At 10 minutes onstream, the conversion was 36% and indicated a large pore zeolite.

### Example 14

The product of Example 7(a) was treated as in Examples 9 and 10. Next, the catalyst was dried at 315°C (600°F), cooled in a closed system and then vacuum impregnated with an aqueous solution of Pd (NH₃)₄ 2 NO₃ to give 0.5 wt.% loading of palladium. The catalyst was then calcined slowly up to 482°C (900°F) in air and held there for three hours. Table 5 gives run conditions and product data for the hydrocracking of hexadecane. The catalyst is quite stable at the temperatures given.

**Table 5**

| | | |
|---|---|---|
| Temp. | 279°C (535°F) | 293°C (560°F) |
| WHSV | 1.55 | 1.55 |
| MPa (PSIG) | 8.27 (1200) | 8.27 1200 |
| Conversion | 94.2 | 99.8 |
| Isom. select. | 83.3 | 17.2 |
| Crack. select. | 16.7 | 82.9 |
| C₅+/C₄ | 18 | 13.3 |
| C₅+C₆/C₅+ | 13.2 | 17.9 |

The data shows that the catalyst has good isomerization selectivity and that the liquid yield is high compared with the gas make.

## Claims

1. A zeolite having a mole ratio of an oxide selected · from silicon oxide, germanium oxide and mixtures thereof to an oxide selected from aluminum oxide, gallium oxide, iron oxide, and mixtures thereof greater than about 100:1, and having the X-ray diffraction lines:-
| 2θ | d/n | I/Iₒ |
|---|---|---|
| 6.10 | 14.49 | 6 |
| 7.38 | 11.98 | 30 |
| 8.18 | 10.81 | 11 |
| 20.30 | 4.37 | 15 |
| 21.12 | 4.21 | 69 |
| 22.25 | 3.99 | 100 |
| 24.73 | 3.60 | 23 |
| 30.90 | 2.89 | 11 |

2. A zeolite having a composition, as synthesized and in the anhydrous state, in terms of mole ratios of oxides as follows:
(0.1 to 10) Q₂O : (0.1 to 5.0) M₂O : W₂O₃: (> 100) YO₂ wherein M is an alkali metal cation, W is selected from aluminum, gallium, iron, and mixtures thereof, Y is selected from silicon, germanium and mixtures thereof, Q is a [5.2.1.0] tricyclodecane quaternary ammonium ion, and having the X-ray diffraction lines as defined in claim 1.

3. The zeolite according to Claim 2 wherein W is aluminum and Y is silicon.

4. A zeolite prepared by thermally treating the zeolite of Claim 2 or 3 at a temperature from about 200°C to 820°C.

5. A zeolite according to Claim 2 wherein the tricyclodecane quaternary ammonium ion is derived from a tricyclodecane compound of the formula: 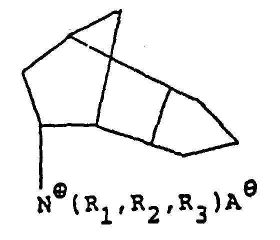 wherein each of R₁, R₂ and R₃ indepepdently is lower alkyl and A^{θ} is an anion which is not detrimental to the formation of the zeolite.

6. A zeolite according to Claim 5 wherein R₁, R₂ and R₃ are the same and each is methyl; and A^{θ} is OH, or I.

7. A zeolite according to any Claim 1 to 6 which has undergone ion exchange with hydrogen, ammonium, rare earth metal, Group IIA metal, or Group VIII metal ions.

8. A zeolite according to any Claim 1 to 7 wherein rare earth metals, Group IIA metals, or Group VIII metals are occluded in the zeolite.

9. A zeolite composition, comprising a zeolite of any Claim 1 to 8, and an inorganic matrix.

10. A method for preparing the zeolite of any Claim 1 to 8, comprising:-
a) preparing an aqueous mixture containing source of an tricyclodecane quaternary ammonium ion, an oxide selected from aluminum oxide, gallium oxide, iron oxide, and mixtures thereof, and an oxide selected from silicon oxide, germanium oxide, and mixtures thereof;
b) maintaining the mixture at a temperature of at least 140°C until the crystals of said zeolite form; and
c) recovering said crystals.

11. The method according to Claim 10 wherein the aqueous mixture has a composition in terms of mole ratios of oxides falling in the ranges: YO₂/W₂O₃ is greater than 50; Q/YO₂ is 0.05:1 to 0.50:1; wherein Y is selected from silicon or germanium and mixtures thereof, W is selected from aluminium, gallium or iron, and mixtures thereof, and Q is an tricyclodecane quaternary ammonium ion.

12. A method according to Claim 11 or 12 wherein the tricyclodecane quaternary ammonium ion is derived from an tricyclodecane compound of the formula: 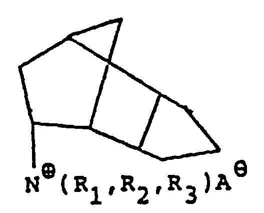 wherein each of R₁, R₂ and R₃ independently is lower alkyl and A^{θ} is an anion which is not detrimental to the formation of the zeolite.

13. A method according to Claim 12 wherein each of R₁, R₂ and R₃ independently is methyl or ethyl; A^{θ} is OH or halogen.

14. A method according to Claim 12 wherein R₁, R₂ and R₃ are the same and each is methyl; and A^{θ} is OH, or I.

15. A process for converting hydrocarbons comprising contacting a hydrocarbonaceous feed at hydrocarbon converting conditions with a zeolite of any Claim 1 to 8.

16. The process of Claim 15 which, is hydrocracking process comprising contacting the hydrocarbon feed-stock under hydrocracking conditions with a zeolite of any Claim 1 to 8.

17. The process of Claim 16 wherein the zeolite contains a hydrogenation component.

18. The process of Claim 15 which is a process for preparing a product having an increased aromatics content comprising:-
a. contacting a hydrocarbonaceous feed, which comprises normal and slightly branched hydrocarbons having a boiling range above 40°C and less than about 200°C under aromatic conversion conditions with a zeolite of any Claim 1 to 8; wherein said zeolite is substantially free of acidity; and
b. recovering an aromatic-containing effluent.

19. The process of Claim 18 wherein the zeolite contains a Group VIII metal component.

20. The process of Claim 15 which is a catalytic cracking process comprising the step of contacting the hydrocarbon feedstock in a reaction zone under catalytic cracking conditions in the absence of added hydrogen with a catalyst comprising a zeolite of any Claim 1 to 8.

21. The process of Claim 15 which is a catalytic cracking process comprising the step of contacting the hydrocarbon feedstock in a reaction zone under catalytic cracking conditions in the absence of added hydrogen with a catalyst composition comprising a component which is a zeolite of any Claim 1 to 8 and a large pore size crystalline aluminosilicate cracking component.

22. The process of Claim 21 wherein the catalyst composition comprises a physical mixture of the two components.

23. The process of Claim 21 or 22 wherein the two catalyst components are incorporated in an inorganic matrix.

24. The process of Claim 15 which is a dewaxing process comprising contacting the hydrocarbon feedstock under dewaxing conditions with a zeolite of any Claim 1 to 8.

25. The process of Claim 22 wherein the zeolite contains a Group VIII or Group VI metal component.

## Patentansprüche

1. Zeolith, in dem das Molverhältnis zwischen einem Oxid, ausgewählt aus Siliciumoxid, Germaniumoxid und Gemischen davon, und einem Oxid, ausgewählt aus Aluminiumoxid, Galliumoxid, Eisenoxid und Gemischen davon, größer ist als etwa 100 zu 1 und der folgende Röntgenbeugungslinien besitzt:
| 2θ | d/n | I/Io |
|---|---|---|
| 6.10 | 14.49 | 6 |
| 7.38 | 11.98 | 30 |
| 8.18 | 10.81 | 11 |
| 20.30 | 4.37 | 15 |
| 21.12 | 4.21 | 69 |
| 22.25 | 3.99 | 100 |
| 24.73 | 3.60 | 23 |
| 30.90 | 2.89 | 11 |

2. Zeolith, dessen Zusammensetzung, wie synthetisiert und im wasserfreien Zustand, bezogen auf die Molverhältnisse der Oxide wie folgt ist:
(0.1 bis 10) Q₂O: (0.1 bis 5.0) M₂O:W₂O₃:(>100) YO₂,
wobei M ein Alkalimetallkation ist, W ausgewählt ist aus Aluminium, Gallium, Eisen und Gemischen davon, Y ausgewählt ist aus Silicium, Germanium und Gemischen davon, Q ein [5,2,1,0]-Tricyclodekan-*quart*.Ammonium-Ion ist und der Röntgenbeugungslinien nach Anspruch 1 besitzt.

3. Zeolith nach Anspruch 2, wobei W Aluminium und Y Silicium ist.

4. Zeolith, hergestellt durch thermisches Behandeln des Zeolithen nach Anspruch 2 oder 3 bei einer Temperatur von etwa 200°C bis 820°C.

5. Zeolith nach Anspruch 2, wobei das Tricyclodekan-quart.Ammonium-Ion abgeleitet ist aus einer Tricyclodekanverbindung der Formel 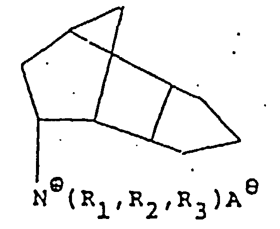 wobei R₁, R₂ und R₃ unabhängig voneinander Niederalkyle sind und A^{θ} ein Anion ist, das die Zeolith-Bildung nicht behindert.

6. Zeolith nach Anspruch 5, wobei R₁, R₂ und R₃ gleich und jeweils Methyl sind und A^{θ} OH oder I ist.

7. Zeolith nach einem der Ansprüche 1 bis 6, der unterworfen wurde einem Ionenaustausch mit Wasserstoff, Ammonium, Seltenerd-Metallen, Metall-Ionen der Gruppe IIA oder VIII.

8. Zeolith nach einem der Ansprüche 1 bis 7, wobei Seltenerd-Metalle, Metalle der Gruppe IIA oder VIII in den Zeolithen eingeschlossen sind.

9. Zeolith-Zusammensetzung, umfassend einen Zeolithen nach einem der Ansprüche 1 bis 8 und eine anorganische Matrix.

10. Verfahren zum Herstellen eines Zeolithen nach einem der Ansprüche 1 bis 8, umfassend
a) Herstellen eines wäßrigen Gemischs, enthaltend eine Tricyclodekan-quart.Ammonium-Ion-quelle, ein Oxid, ausgewählt aus Aluminiumoxid, Galliumoxid, Eisenoxid und Gemischen davon, und ein Oxid, ausgewählt aus Siliciumoxid, Germaniumoxid und Gemischen davon;
b) Halten des Gemischs bei einer Temperatur von mindestens 140°C bis die Zeolith-Kristalle gebildet sind; und
c) Gewinnen der Kristalle.

11. Verfahren nach Anspruch 10, wobei das wäßrige Gemisch eine Zusammensetzung hat, bezogen auf die Molverhältnisse der Oxide, in den Bereichen: YO₂/W₂O₃ größer als 50; Q/YO₂ gleich 0.05:1 bis 0.50:1; wobei Y ausgewählt ist aus Silicium oder Germanium und Gemischen davon, W ausgewählt ist aus Aluminium, Gallium oder Eisen und Gemischen davon, und Q ein Tricyclodekan-quart.Ammonium-Ion ist.

12. Verfahren nach Anspruch 11 oder 12, wobei das Tricyclodekan-quart.Ammonium-Ion abgeleitet ist aus einer Tricylodekanverbindung der Formel: 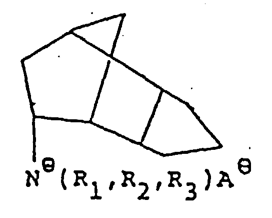 wobei R₁, R₂ und R₃ unabhängig voneinander Niederalkyle sind und A^{θ} ein Anion ist, das die Zeolith-Bildung nicht behindert.

13. Verfahren nach Anspruch 12, wobei R₁, R₂ und R₃ unabhängig voneinander Methyl oder Ethyl sind, A^{θ} OH oder Halogen ist.

14. Verfahren nach Anspruch 12, wobei R₁, R₂ und R₃ gleich und jeweils Methyl sind und A^{θ} OH oder I ist.

15. Verfahren zur Umwandlung von Kohlenwasserstoffen, umfassend das Kontaktieren eines Kohlenwasserstoff-Ausgangsmaterials mit einem Zeolithen nach einem der Ansprüche 1 bis 8 unter Kohlenwasserstoff-Umwandlungsbedingungen.

16. Verfahren nach Anspruch 15, das ein Hydrocrackverfahren ist, umfassend das Kontaktieren der Kohlenwasserstoff-Ausgangsmaterialien mit einem Zeolithen nach einem der Ansprüche 1 bis 8 unter Hydrocrackbedingungen.

17. Verfahren nach Anspruch 16, wobei der Zeolith eine Hydrierungskomponente enthält.

18. Verfahren nach Anspruch 15, das ein Verfahren ist zum Herstellen eines Produkts mit erhöhtem Aromatenanteil, umfassend
a) Kontaktieren eines Kohlenwasserstoff-Ausgangsmaterials, das umfaßt normale und leichtverzweigte Kohlenwasserstoffe mit Siedepunkten über 40°C und unter etwa 200°C, unter Aromaten-Umwandlungsbedingungen mit einem Zeoliten einer der Ansprüche 1 bis 8, wobei der Zeolith im wesentlichen säurefrei ist; und
b) Zurückgewinnen des Aromaten-haltigen Abwassers.

19. Verfahren nach Anspruch 18, wobei der Zeolith eine Metallkomponente der Gruppe VIII enthält.

20. Verfahren nach Anspruch 15, das ein katalytisches Crackverfahren ist, umfassend den Kontaktierungsschritt des Kohlenwasserstoff-Ausgangsmaterials in einer Reaktionszone unter katalytischen Crackbedingungen in Abwesenheit von zugesetztem Wasserstoff mit einem Katalysator, umfassend einen Zeolithen nach einem der Ansprüche 1 bis 8.

21. Verfahren nach Anspruch 15, das ein katalytisches Crackverfahren ist, umfassend den Kontaktierungsschritt des Kohlenwasserstoff-Ausgangsmaterials in einer Reaktionszone unter katalytischen Crackbedingungen in Abwesenheit von zugesetztem Wasserstoff mit einer Katalysatorzusammensetzung, umfassend eine Komponente, die ein Zeolith nach einem der Ansprüche 1 bis 8 ist und eine großporige kristalline Aluminiumsilicat-Crackkomponente.

22. Verfahren nach Anspruch 21, wobei die Katalysatorzusammensetzung ein physikalisches Gemisch der zwei Komponenten umfaßt.

23. Verfahren nach Anspruch 21 oder 22, wobei die Katalysatorkomponenten in eine anorganische Matrix eingefügt sind.

24. Verfahren nach Anspruch 15, das ein Entwachsungsverfahren ist, umfassend das Kontaktieren des Kohlenwasserstoff-Ausgangsmaterials mit einem Zeolithen nach einem der Ansprüche 1 bis 8 unter Entwachsungs-Bedingungen.

25. Verfahren nach Anspruch 22, wobei der Zeolith eine Metallkomponente der Gruppe VIII oder VI enthält.

## Revendications

1. Zéolite ayant un rapport molaire d'un oxyde choisi entre l'oxyde de silicium, l'oxyde de germanium et leurs mélanges à un oxyde choisi entre l'oxyde d'aluminium, l'oxyde de gallium, l'oxyde de fer et leurs mélanges supérieur à environ 100:1, et comportant les raies de diffraction des rayons X :
| 2θ | d/n | I/Iₒ |
|---|---|---|
| 6,10 | 14,49 | 6 |
| 7,38 | 11,98 | 30 |
| 8,18 | 10,81 | 11 |
| 20,30 | 4,37 | 15 |
| 21,12 | 4,21 | 69 |
| 22,25 | 3,99 | 100 |
| 24,73 | 3,60 | 23 |
| 30,90 | 2,89 | 11 |

2. Zéolite ayant une composition, telle qu'elle est obtenue par synthèse et à l'état anhydride, en termes des rapports molaires des oxydes, comme suit :
(0,1 à 10) Q₂O : (0,1 à 5,0) M₂O : W₂O₃ : (> 100) YO₂ dans laquelle M représente un cation de métal alcalin, W est choisi entre l'aluminium, le gallium, le fer et leurs mélanges, Y est choisi entre le silicium, le germanium et leurs mélanges, Q représente un ion [5.2.1.0]-tricyclodécane-ammonium quaternaire, et comportant les raies de diffraction des rayons X répondant à la définition suivant la revendication 1.

3. Zéolite suivant la revendication 2, dans laquelle W représente l'aluminium et Y représente le silicium.

4. Zéolite préparée par traitement thermique de la zéolite suivant la revendication 2 ou 3 à une température d'environ 200°C à 820°C.

5. Zéolite suivant la revendication 2, dans laquelle l'ion tricyclodécane-ammonium quaternaire est dérivé d'un tricyclodécane de formule : 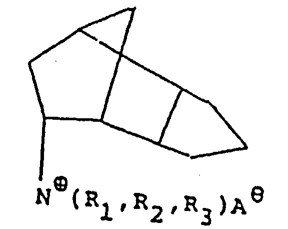 dans laquelle chacun des groupes R₁, R₂ et R₃ représente, indépendamment, un groupe alkyle inférieur et A^{θ} représente un anion qui n'est pas néfaste pour la formation de la zéolite.

6. Zéolite suivant la revendication 5, dans laquelle R₁, R₂ et R₃ sont identiques et représentent chacun un groupe méthyle ; et A^{θ} représente un groupe OH ou I.

7. Zéolite suivant l'une quelconque des revendications 1 à 6, qui a subi un échange d'ions avec des ions hydrogène, des ions ammonium, des ions de métaux faisant partie des terres rares, des ions de métaux du Groupe IIA ou des ions de métaux du Groupe VIII.

8. Zéolite suivant l'une quelconque des revendications 1 à 7, dans laquelle les métaux faisant partie des terres rares, les métaux du Groupe IIA ou les métaux du Groupe VIII sont occlus dans la zéolite.

9. Composition zéolitique, comprenant une zéolite suivant l'une quelconque des revendications 1 à 8 et une matrice inorganique.

10. Procédé pour la préparation de la zéolite suivant l'une quelconque des revendications 1 à 8, comprenant les étapes consistant :
a) à préparer un mélange aqueux contenant une source d'un ion tricyclodécane-ammonium quaternaire, un oxyde choisi entre l'oxyde d'aluminium, l'oxyde de gallium, l'oxyde de fer et leurs mélanges, et un oxyde choisi entre l'oxyde de silicium, l'oxyde de germanium et leurs mélanges ;
b) à maintenir le mélange à une température d'au moins 140°C jusqu'à la formation des cristaux de ladite zéolite ; et
c) à recueillir lesdits cristaux.

11. Procédé suivant la revendication 10, dans lequel le mélange aqueux a une composition, en termes des rapports molaires des oxydes, comprise dans les intervalles suivants : YO₂/W₂O₃ est supérieur à 50 ; Q/YO₂ est compris dans l'intervalle de 0,05:1 à 0,50:1 ; rapports dans lesquels Y est choisi entre le silicium, le germanium et leurs mélanges ; W est choisi entre l'aluminium, le gallium, le fer et leurs mélanges, et Q représente un ion tricyclodécane-ammonium quaternaire.

12. Procédé suivant la revendication 11 ou 12, dans lequel l'ion tricyclodécane-ammonium quaternaire est dérivé d'un tricyclodécane de formule : 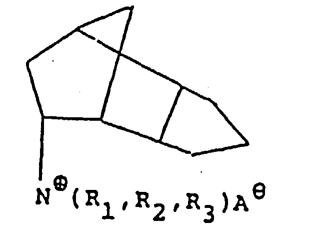 dans laquelle chacun des groupes R₁, R₂ et R₃ représente, indépendamment, un groupe alkyle inférieur et A^{θ} représente un anion qui n'est pas néfaste pour la formation de la zéolite.

13. Procédé suivant la revendication 12, dans lequel chacun des groupes R₁, R₂ et R₃ représente, indépendamment, un groupe méthyle ou éthyle ; A^{θ} représente un groupe OH ou un halogène.

14. Procédé suivant la revendication 12, dans lequel R₁, R₂ et R₃ sont identiques et représentent chacun un groupe méthyle ; et A^{θ} représente un groupe OH ou I.

15. Procédé de conversion d'hydrocarbures, comprenant la mise en contact d'une charge hydrocarbonée dans des conditions de conversion d'hydrocarbures avec une zéolite suivant l'une quelconque des revendications 1 à 8.

16. Procédé suivant la revendication 15, qui est un procédé d'hydrocraquage comprenant la mise en contact de la charge hydrocarbonée d'alimentation dans des conditions d'hydrocraquage avec une zéolite suivant l'une quelconque des revendications 1 à 8.

17. Procédé suivant la revendication 16, dans lequel la zéolite contient un constituant d'hydrogénation.

18. Procédé suivant la revendication 15, qui est un procédé pour la préparation d'un produit ayant une teneur accrue en composés aromatiques, comprenant les étapes consistant :
a. à mettre en contact une charge hydrocarbonée, qui comprend des hydrocarbures normaux et des hydrocarbures légèrement ramifiés ayant une plage d'ébullition supérieure à 40°C et inférieure à environ 200°C dans des conditions de conversion de composés aromatiques avec une zéolite suivant l'une quelconque des revendications 1 à 8 ; ladite zéolite étant pratiquement dépourvue d'acidité ; et
b. à recueillir un effluent contenant les composés aromatiques.

19. Procédé suivant la revendication 18, dans lequel la zéolite contient un constituant qui est un métal du Groupe VIII.

20. Procédé suivant la revendication 15, qui est un procédé de craquage catalytique comprenant l'étape de mise en contact de la charge d'hydrocarbures d'alimentation dans une zone réactionnelle dans des conditions de craquage catalytique en l'absence d'hydrogène ajouté, avec un catalyseur comprenant une zéolite suivant l'une quelconque des revendications 1 à 8.

21. Procédé suivant la revendication 15, qui est un procédé de craquage catalytique, comprenant l'étape de mise en contact de la charge d'hydrocarbures d'alimentation dans une zone réactionnelle dans des conditions de craquage catalytique en l'absence d'hydrogène ajouté, avec une composition de catalyseur comprenant un constituant qui est une zéolite suivant l'une quelconque des revendications 1 à 8 et un constituant de craquage qui est un aluminosilicate cristallin à pores de grand diamètre.

22. Procédé suivant la revendication 21, dans lequel la composition de catalyseur comprend un mélange physique des deux constituants.

23. Procédé suivant la revendication 21 ou 22, dans lequel les deux constituants de catalyseur sont incorporés à une matrice inorganique.

24. Procédé suivant la revendication 15, qui est un procédé de déparaffinage comprenant la mise en contact de la charge d'hydrocarbures d'alimentation dans des conditions de déparaffinage avec une zéolite suivant l'une quelconque des revendications 1 à 8.

25. Procédé suivant la revendication 22, dans lequel la zéolite contient un constituant qui est un métal du Groupe VIII ou du Groupe VI.
